# EUROPÄISCHE PATENTANMELDUNG

(11) **EP 0 778 017 A2**
(43) Veröffentlichungstag der Anmeldung: **11.06.1997**
(21) Anmeldenummer: 96119713.4
(22) Anmeldetag: 09.12.1996
(51) Int. Cl.: A61H 39/00

(54) **Akupunkturkissen**

(30) Priorität: 09.12.1995 DE 19952019551U
(71) Anmelder: Bonke, Christoph, Dr., 83126 Flintsbach (DE)
(72) Erfinder: Bonke, Christoph, Dr., 83126 Flintsbach (DE)

(57) **Zusammenfassung**

Die vorliegende Erfindung betrifft ein Kopfkissen aus nicht wesentlich verformbarem Material mit einer mittleren muldenförmigen Aussparung (1) zur Aufnahme und Abstützung des Kopfes bei Rückenlage und/oder Bauchlage und/oder Seitenlage, welche im wesentlichen entsprechend der Anatomie des Hinterkopfes und/oder der Gesichtshälfte und/oder des seitlichen Kopfbereiches geformt ist. Auf der Oberfläche der muldenförmigen Aussparung (1) und/oder im Bereich der Nacken- beziehungsweise Kinnauflage (2) und/oder auf der Oberseite mindestens eines Seitenteils (18) des Kissens (6) ist mindestens eine feststehende Stimulationsvorrichtung (3) zur Ausübung von physikalischen Reizen wie von magnetischen Reizen und/oder elektrischen Reizen und/oder Druckreizen und/oder thermischen Reizen und/oder Lichtreizen vorgesehen.

## Beschreibung

Die vorliegende Erfindung betrifft ein Kissen zum Auflegen des Kopfes in Bauch- und/oder Rückenlage, welches vornehmlich im Rahmen einer Behandlung nach der Akupunktur- beziehungsweise der Akupressurlehre zur Verabreichung von physikalischen Reizen geeignet ist, insbesondere von magnetischen, elektrischen oder thermischen Reizen sowie von Druck- und/oder Lichtreizen.

In der zu erwartenden Novelle der deutschen Gebührenordnung für Äzte (GOÄ) ist die Akupunktur offiziell mit eigenständigen Abrechnungsziffern vorgesehen. Die Lehre der Akupunktur ist demnach heutzutage allgemein anerkannt.

Die klassische Nadel-Akupunktur verwendet Einstiche mit Gold- und Silbernadeln an lehrmäßig genau festgelegten Hautpunkten, die spontan- oder druckschmerzhaft sein können, bei funktionellen, reversiblen Erkrankungen und Störungen zu diagnostischen und/oder therapeutischen Zwecken.

Bei dieser Nadelakupunktur kommt es gelegentlich zu Zwischenfällen.

Beispielsweise empfinden Patienten vor dem ersten Einstich Angst, die in Verkrampfungen ihren Ausdruck finden kann oder bei stehender Behandlung sogar zu einem Kollaps führen kann.

Sehr häufig begegnet man dem Phänomen, daß die gesetzten Nadeln vorzeitig abfallen. Meist als Folge einer Verkrampfung, hervorgerufen durch psychische Verspannung oder physisch durch das längere Verharren in einer manchmal nicht bequemen Position, werden die Nadeln früher ausgestoßen, als dies zu erwarten wäre. Insbesondere beim Gehen vom Behandlungszimmer zum Ruheraum kommt es häufig zum Abfallen der Nadeln.
Nach der herrschenden Meinung sind die abgefallenen Nadeln pro Sitzung bis zu zweimal zu ersetzen. Die Anzahl der in der Regel als unangenehm empfundenen Nadeleinstiche wird dadurch vervielfacht und erzeugt nicht nur bei Kindern eine ablehnenden Haltung gegenüber der Akupunktur.

Bei am Kopf gesetzten Nadeln sollte der Patient in der ersten Phase auch nicht sprechen, da durch die damit verbundenen Bewegungen der mimischen Muskulatur die Nadeln in Schwingung kommen und aufgrund dieser mechanischen Ursache abfallen können. Insbesondere bei Kindern stellt das gegebenenfalls längerfristige Sprechverbot ein Problem dar.

Gerade im Falle von lebhaften Kindern ist es ferner äußerst problematisch, diese über den langen Zeitraum einer Sitzung mit im Kopfbereich gesetzten Nadeln ruhig zu halten.

Ein weiterer schwerwiegender Nachteil der Nadelakupunktur liegt darin, daß der Patient für die Anfahrt zu dem Behandler, die dort abzuwartende Wartezeit und die Behandlung selbst in der Regel mehrere Stunden seiner wertvollen Tages-Arbeitszeit verliert.

Nachteilig ist unter dem Aspekt eines Zeitverlustes weiterhin, daß der Behandler zu Beginn jeder Sitzung die jeweils anzusprechenden Akupunkturpunkte neu auffinden muß.

Gelegentlich finden sich Überempfindlichkeitsreaktionen gegenüber in die Haut eindringende Nadeln, insbesondere auf Silbernadeln.

Wird die Nadelakupunktur mit unsterilen Akupunkturnadeln durchgeführt, kommt es regelmäßig zu Entzündungen der Einstichstelle.

Vor dem Hintergrund, daß die Nadelakupunktur nicht ausschließlich von medizinisch geschultem Personal durchgeführt wird, stellt die von bereits benutzten Akupunkturnadeln ausgehende Infektionsgefahr einen erheblichen Nachteil der Nadel-Akupunktur dar. Die Übertragung von Krankheiten wie AIDS und Hepatitis ist auf diese Weise möglich.

Es ist ferner bekannt, anstelle von langen Akupunkturnadeln kurze Nadeln zu verwenden, die an ihrem rückwärtigen Ende eine tellerförmige Verbreiterung aufweisen, welche an der klebenden Seite eines kreisförmigen Pflasters aufliegt. Wird das Pflaster auf die Haut aufgeklebt, dringt die kurze Nadel in die Haut ein und wird durch das auf der Haut haftende Pflaster an einem vorzeitigen Abfallen gehindert.

Wird ein derartiges Nadelpflaster beispielsweise im Bereich des Hinterkopfes angebracht, ist die Entfernung des Pflasters entweder mit einem schmerzhaften Ziehen an den Haaren oder mit dem Einsatz umwelt- und hautschädigender Lösungsmittel verbunden.

Alternativ hierzu bietet sich die Möglichkeit des Ausrasierens der zu akupunktierenden Stelle an. Gerade bei Trägern längeren Haares erfreut sich dieses Vorgehen jedoch keiner Beliebtheit.

Da weder das Tragen eines Pflasters am Hinterkopf noch ein punktförmiges Ausrasieren des Haupthaares im Hinterkopf- oder Schläfenbereich ästhetisch besonders ansprechend ist, läßt die Akzeptanz derartiger Nadelpflaster zu wünschen übrig.

Von besonderem Nachteil ist ferner, daß derartige Nadelpflaster während des Umdrehens im Schlafe leicht abgelöst werden.
Einerseits geht von der Nadel des auf diese Weise abgelösten Nadelpflasters eine Verletzungsgefahr aus. Andererseits wird die Bettwäsche durch den Pflasterklebstoff leicht verschmutzt.

Darüberhinaus geht auch der Einsatz eines Nadelpflasters bei einer längeren Verweildauer der Nadel in der Haut oftmals mit Entzündungen der Einstichstelle einher.

Unverträglichkeitsreaktionen der Haut mit dem Klebstoff solcher Pflaster werden außerdem beobachtet.

Aufgabe der vorliegenden Erfindung ist daher die Bereitstellung eines Kissens zum Auflegen des Kopfes in Bauchund/oder Rücken- und/oder Seitenlage, welches zur Verabreichung von physikalischen Reizen auf definierte Akupunkturpunkte der Haut geeignet ist, dessen Anlegen und Betreiben nicht mit einem Schmerzerlebnis des Benutzers verbunden ist, das sich aufgrund seiner Bequemlichkeit und Schmerzfreiheit bei Kindern und Erwachsenen einer großen Akzeptanz erfreut, das der Entstehung von Angstzuständen und Kollapssituationen während der Verabreichung von physikalischen Reizen entgegenwirkt, die Unterbrechung der Sitzung durch abfallende Reizapplikatoren ausschließt, selbst während der Behandlung das Sprechen erlaubt, durch in die Haut eindringende Reizapplikatoren verursachte Überempfindlichkeiten vermeidet, die von unsterilen, infizierten, in die Haut eindringenden Reizapplikatoren ausgehende Entzündungs- beziehungsweise Infektionsgefahr nicht kennt, selbst im Falle mehrerer Sitzungen lediglich ein einmaliges Aufsuchen der anzusprechenden Akupunkturpunkte erfordert, den Benutzer in die Lage versetzt, auch außerhalb der Sprechstundenzeit seines Behandlers und ohne die Zeit für eine Anfahrt dorthin in Kauf nehmen zu müssen, sich selbst an definierten Akupunkturpunkten physikalische Reize zuzuführen, das eine Verringerung der von den Krankenkassen zu erstattenden Behandlungskosten bewirkt, dessen Trennung vom Kopf nicht schmerzhaft ist und kein Lösungsmittel erfordert, von dem keine Verletzungs- oder Verunreinigungsgefahr während des Schlafes ausgeht und welches zur Verabreichung physikalischer Reize auf den Hinterkopf nicht eines punktförmigen Ausrasierens des Haupthaares oder des Anbringens entstellender Pflaster auf diesem bedarf.

Erfindungsgemäß wird diese Aufgabe bei einem gattungsgemäßen Kissen durch die im kennzeichnenden Teil des Patentanspruchs 1 angegebenen Merkmale gelöst.
Besonders bevorzugte Ausführungsformen sind Gegenstand der Unteransprüche.

Ausführungsbeispiele der vorliegenden Erfindung werden anhand der Zeichnungen näher beschrieben.
Es zeigen:
Abbildung 1 eine frontale perspektivische Ansicht eines erfindungsgemäßen Kissens von schräg oben;

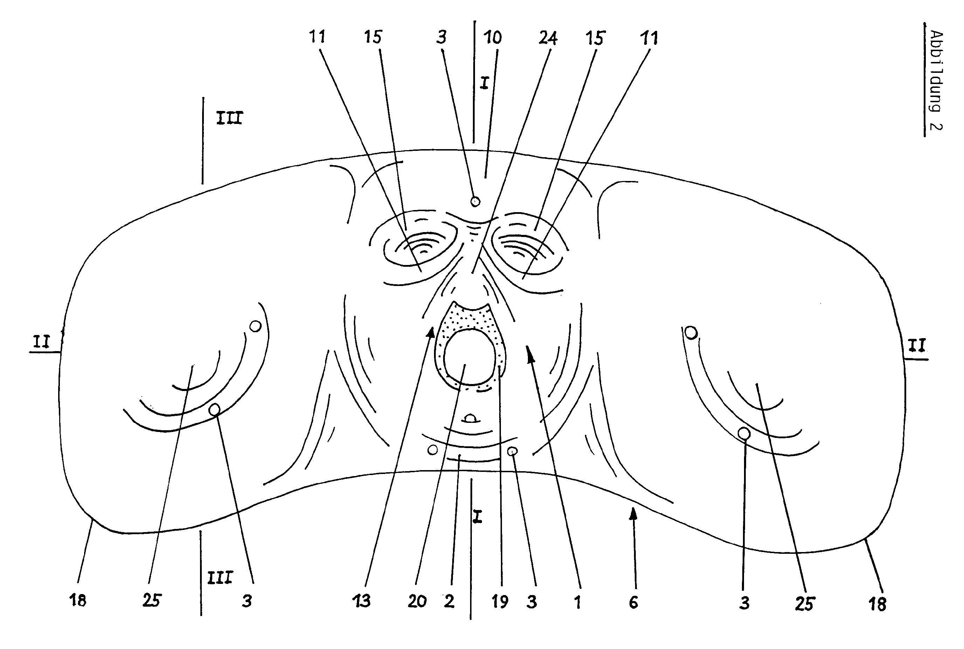

Abbildung 2 eine Draufsicht auf ein erfindungsgemäßes Kissen;

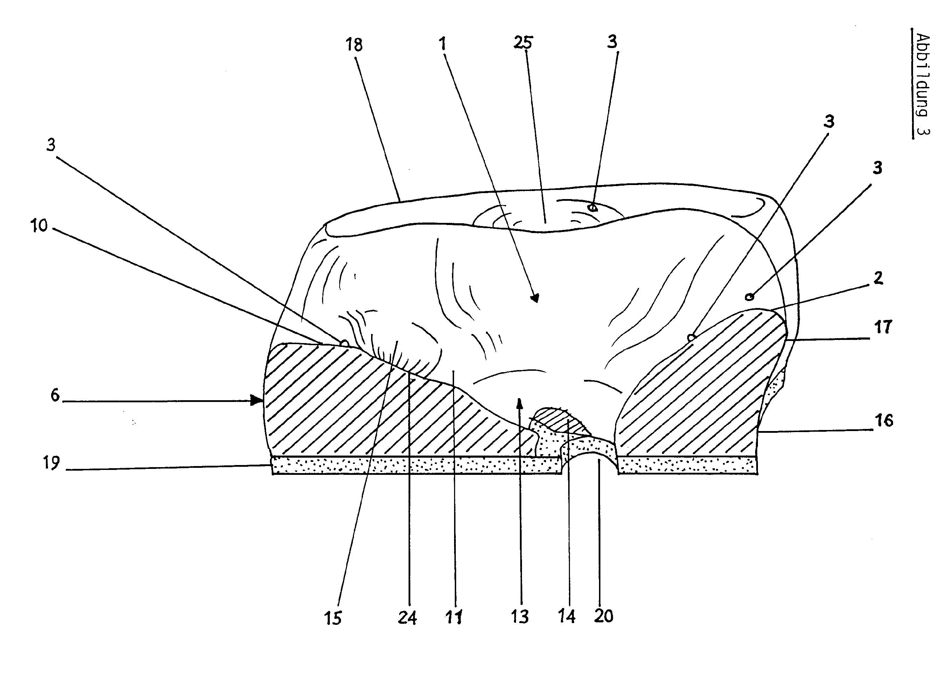

Abbildung 3 einen Querschnitt eines erfindungsgemäßen Kissens entlang der Linie I - I in Abbildung 2;

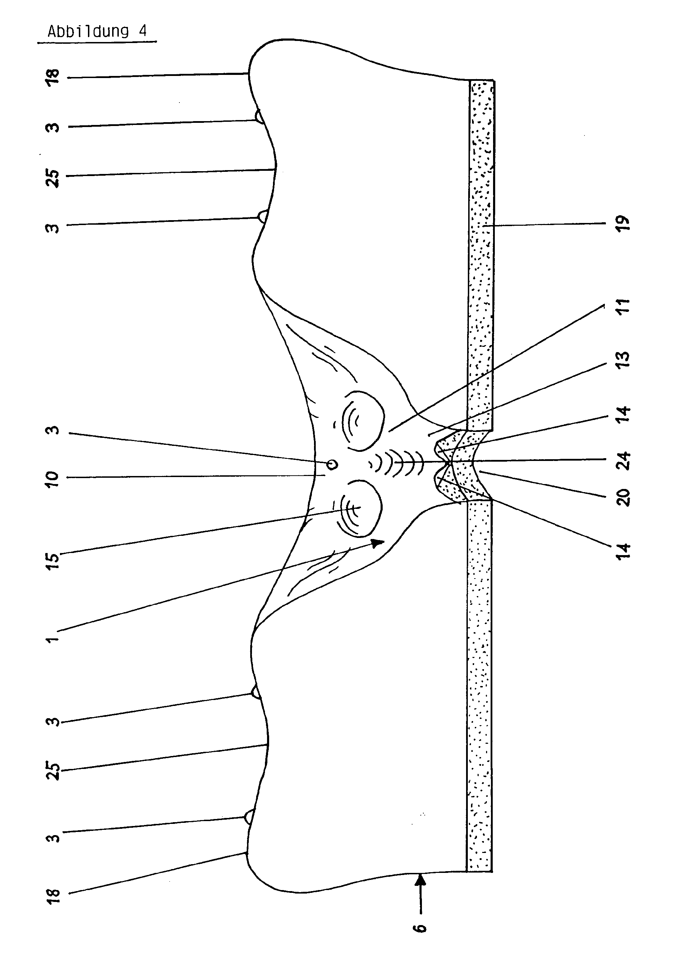

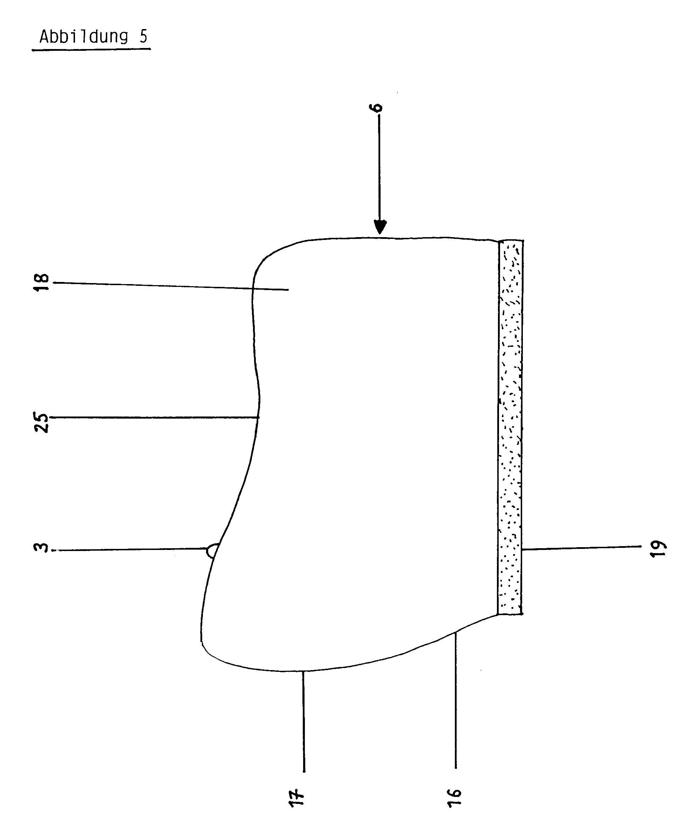

Abbildung 4 einen Längsschnitt eines erfindungsgemäßen Kissens entlang der Linie II - II in Abbildung 2;
Abblidung 5 einen Querschnitt eines Seitenteils eines erfindungsgemäßen Kissens entlang der Linie III - III in Abbildung 2;
Abbildung 6 eine schematische Ansicht eines menschlichen Kopfes von vorne, in der diejenigen Orte (Akupunkturpunkte) durch Punkte markiert sind, auf die mittels eines erfindungsgemäßen Kissens physikalische Reize ausgeübt werden können;
Abbildung 7 eine schematische Ansicht eines menschlichen Kopfes von hinten, in der diejenigen Orte (Akupunkturpunkte) durch Punkte markiert sind, auf die mittels eines erfindungsgemäßen Kissens physikalische Reize ausgeübt werden können;
Abbildung 8 eine schematische Ansicht eines menschlichen Kopfes von der Seite, in der diejenigen Orte (Akupunkturpunkte) durch Punkte markiert sind, auf die mittels eines erfindungsgemäßen Kissens physikalische Reize ausgeübt werden können;

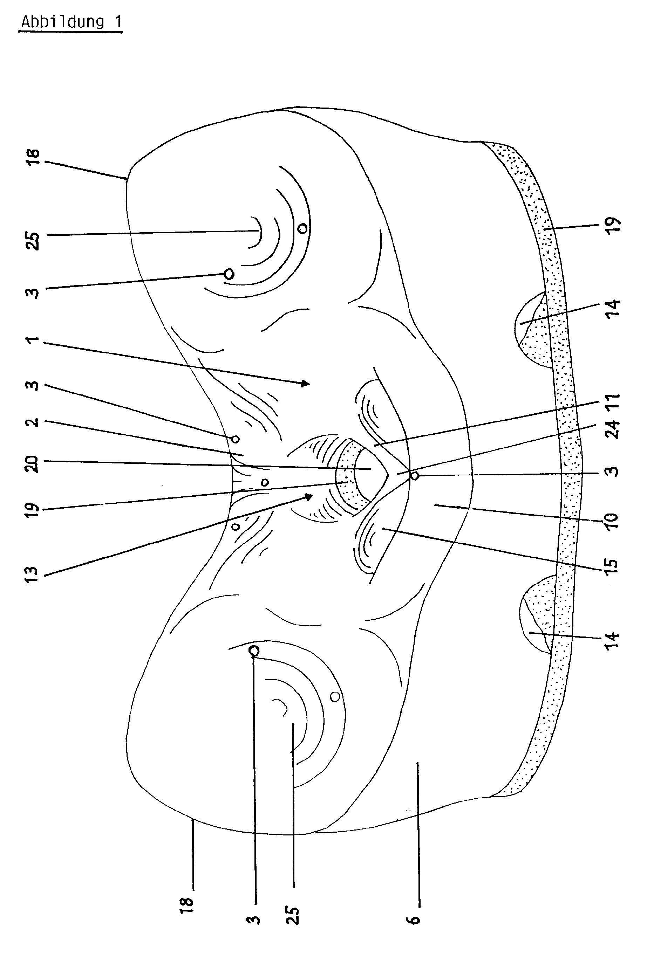

Abbildung 1 zeigt eine frontale Draufsicht auf ein erfindungsgemäßes Kissen von schräg oben. Die rückwärtige, dem Rücken des Benutzers zugewandte Seite des erfindungsgemäßen Kissens zeigt nach hinten, während die scheitelwärtige Seite des Kissens nach vorne in Richtung des Betrachters weist.

Aus Abbildung 1 geht hervor, daß ein erfindungsgemäßes Kissen (6) insbesondere eine mittlere, muldenförmige Aussparung (1) zur Aufnahme und Abstützung des Kopfes bei Rückenlage und/oder Bauchlage und/oder Seitenlage des Benutzers aufweist.
Die mittlere muldenförmige Aussparung (1) ist in besonders bevorzugten Ausführungsformen im wesentlichen entsprechend der Anatomie des Hinterkopfes und/oder der Gesichtshälfte und/oder des seitlichen Kopfbereiches geformt. Durch die Verwendung eines zumindest etwas elastischen, anformbaren Materials im Bereich der Kopfauflagefläche ist sichergestellt, daß die Wandungen der muldenförmigen Aussparung (1) sich den Konturen des Kopfes anschmiegen und mit diesem zumindest teilweise in Kontakt stehen.

Die in Abbildung 1 dargestellte mittlere, muldenförmige Aussparung (1) ist zur Erhöhung des Liegekomforts in Richtung des Scheitels einer auf dem Kissen ruhenden Person nicht besonders weit hochgezogen. Gerade wenn das Kissen insbesondere zu therapeutischen Zwecken verwendet wird, empfiehlt sich jedoch eine sehr weit hochgezogene, mützenähnliche Ausbildung des scheitelseitigen Randbereichs der mittleren, muldenförmigen Aussparung (1). Denn hierdurch vergrößert sich die durch das erfindungsgemäße Kissen zugängliche Kopffläche beträchtlich.

Der in Richtung des Rückens eines Benutzers weisende, obere Randabschnitt der mittleren, muldenförmigen Aussparung (1) ist in der Regel besonders weit nach oben gezogen und steht vorzugsweise in Form einer Nackenauflage (2) mit dem Nacken des Benutzers in Kontakt.
Bei einer Benutzung des erfindungsgemäßen Kissens (6) in der Bauchlage kommt diesem, in Richtung des Rückens eines Benutzers weisenden, oberen Randabschnitt (2) der mittleren muldenförmigen Aussparung (1) überdies die Funktion einer Kinnauflage zu.

Auf der Oberfläche der mittleren, muldenförmigen Aussparung (1), insbesondere auch auf den Oberflächen deren frontseitiger, rückenseitiger und seitlichen Ränder sowie der gegebenenfalls vorhandenen Seitenteile (18) des Kissens (6), kann eine einzelne Stimulationsvorrichtung (3) oder eine Vielzahl hiervon vorgesehen werden. Die Stimulationsvorrichtungen (3) dienen zur Ausübung von physikalischen Reizen jeglicher Art vornehmlich auf den Kopf- und Halsbereich. Insbesondere vermitteln sie magnetische Reize und/oder elektrische Reize und/oder Druckreize und/oder thermische Reize und/oder Lichtreize.

Die Stimulationsvorrichtungen (3) sind vorzugsweise feststehend auf der Oberfläche des Kissens vorgesehen. Dies bedeutet, daß sie ihre Position während einer Behandlungssitzung nicht verändern und nicht beispielsweise an der Spitze von kreisenden Massagefingern angebracht sind.

Selbstverständlich ist es jedoch möglich, die Positionen der Stimulationsvorrichtungen (3) zwischen zwei Sitzungen beispielsweise durch ein Umstecken oder versetztes Aufkleben zu verändern.
Hierdurch wird das erfindungsgemäße Kissen für mehrere Benutzer sowie für eine große Anzahl von Indikationen verwendbar.

Bei den Stimulationsvorrichtungen (3) handelt es sich beispielsweise um magnetische Elemente, die gegenüber der Oberfläche des Kissens erhaben oder in das Kissen integriert sind.
Ist das magnetische Element gegenüber der Oberfläche des Kissens zumindest etwas erhaben, so führt dies zu dem Vorteil, daß es bereits durch geringste Bewegungen des Kopfes, beispielsweise beim Sprechen oder während des Schlafes, zu einer unterstützenden, besonders sanften Massage-Stimulation der Kopf-, Hals- oder Gesichtshaut kommt. Dies gilt entsprechend für die weiter unten beschriebenen, anstelle eines Magneten verwendbaren Stimulationsvorrichtungen (3).

Die Form der verwendbaren Magneten ist vorzugsweise in Richtung des Kopfes flach, abgerundet, spitz oder nadelförmig.
Die magnetische Feldstärke der Magneten liegt im Bereich von 300 bis 2000 Gauß. In der Regel beträgt sie 400 bis 1000 Gauß, vorzugsweise 500 bis 900 Gauß, insbesondere 600 bis 800 Gauß.

Bei den Magneten handelt es sich beispielsweise um Permanentmagneten oder um stromdurchflossene Spulen mit Eisenkern, die kugelförmig, knopfförmig, kreisförmig, oval, länglich oder stabförmig sind. Sie umfassen beispielsweise Ferrit, nickel-, kobalt- und/oder eisenhaltige Stoffe oder keramische oder metallische Magnetwerkstoffe.

Vorzugsweise finden sogenannte Taiki-Magnete aus Ferrit mit einer magnetischen Feldstärke von 700 Gauß Verwendung.

Alternativ zu den Magneten oder zusätzlich zu diesen können als Stimulationsvorrichtungen (3) ein oder mehrere im wesentlichen kugelförmige Druckelemente zur Anwendung kommen. Diese sind in Richtung des Kopfes beispielsweise spitz, nadelförmig oder abgerundet und überragen die Oberfläche des Kissens (6) zumindest etwas oder sind in dieses integriert. Während des Aufliegens des Kopfes üben sie einen im wesentlichen punktförmigen Druck auf diesen und/oder den Halsbereich aus.

Zur Erzeugung von punktförmigen Drücken sind darüberhinaus einen scharf gebündelten Wasserstrahl oder Luftstrahl erzeugende Vorrichtungen geeignet.
Auch Vorrichtungen zur Erzeugung von Schallwellen, insbesondere im hörbaren Bereich oder im Ultraschallbereich, sind unter dem Begriff "Druckelemente" zu verstehen.

Als Stimulationsvorrichtungen (3) kommen ferner in Richtung des Kopfes abgerundete, spitze oder nadelförmige Laserstrahlen ausstrahlende Vorrichtungen in Betracht, welche die Oberfläche des Kissens (6) überragen oder in dieses integriert sind. Während des Aufliegens des Kopfes üben sie im wesentlichen punktförmige Licht- und/oder thermische Reize auf diesen und/oder den Halsbereich aus. Bei diesen Laserstrahlen ausstrahlenden Vorrichtungen handelt es sich beispielsweise um Enden von Glasfaserlichtleitern, um Laserdioden oder um andere punktförmige Laserquellen.

Die emittierten Laserstrahlen verfügen lediglich über eine geringe Energie und werden als Softlaserstrahlen bezeichnet. Ihnen kommt insbesondere eine lichtreizende und membranstabilisierende Funktion zu. Zu ihrer Erzeugung dienen handelsübliche Lasergeräte.

Das erfindungsgemäße Kissen ist insbesondere auch zur Anwendung im Rahmen einer Farbtherapie geeignet. Zu diesem Zwecke können als Stimulationsvorrichtungen (3) in Richtung des Kopfes abgerundete, spitze oder nadelförmige farbiges Licht ausstrahlende Vorrichtungen vorgesehen werde, welche die Oberfläche des Kissens überragen oder in dieses integriert sind. Diese Stimulationsvorrichtungen (3) üben während des Aufliegens des Kopfes im wesentlichen punktförmige Lichtreize auf diesen und/oder den Halsbereich aus.

Insbesondere Leuchtdioden, Enden von gegebenenfalls flexiblen Lichtleitern oder kleine Glühlampen mit Farbfilter-Vorsätzen sind hierfür verwendbar.

Als Stimulationsvorrichtung (3) eignet sich beispielsweise auch ein Heiz- und/oder Kühlelement, welches die Oberfläche des Kissens (6) überragt oder in dieses integriert ist und während des Aufliegens des Kopfes eine punktuelle Erwärmung oder Abkühlung bewirkt. Das Heizelement ist beispielsweise ein stromdurchflossener Widerstandsdraht, das Kühlelement beispielsweise eine Peltier-Zelle. Selbstverständlich sind alternativ hierzu Geräte zur Erzeugung eines temperierten Wasser- oder Luftstrahles verwendbar.

Die Stimulationsvorrichtung (3) kann ferner beispielsweise durch eine kleine Elektrode verkörpert werden, welche die Oberfläche des Kissens (6) überragt oder in dieses integriert ist und während des Aufliegens des Kopfes punktuell niederfrequente Wechselströme zu diesem weiterleitet. Zur Erzeugung der niederfrequenten Wechselspannung sind handelsübliche, insbesondere in der Elektrotherapie oder der Elektroakupunktur verwendete Reizimpulsgeneratoren geeignet.

Selbstverständlich ist es möglich, nicht nur eine einzelne Stimulationsvorrichtung (3) auf der Oberfläche der mittleren, muldenförmigen Aussparung (1) oder des Seitenteils (18) vorzusehen, sondern eine Vielzahl von diesen. Dabei können alle eingesetzten Stimulationsvorrichtungen vom gleichen Typ sein oder voneinander verschiedene Stimulationsvorrichtungen sein. So kann die Gesamtheit aller auf dem Kissen vorgesehenen Stimulationsvorrichtungen (3) eine Kombination von einem oder mehreren Magneten, Druckelementen, Heiz- und/oder Kühlelementen, Elektroden und Vorrichtungen zur Ausstrahlung von Laser- und farbigen Lichtstrahlen sein.

Ein wesentliches Merkmal des erfindungsgemäßen Kissens besteht ferner darin, daß die Stimulationsvorrichtungen (3) in der mittleren, muldenförmigen Aussparung (1) und/oder in der Nacken- beziehungsweise Kinnauflage (2) und/oder auf mindestens einem Seitenteil (18) jeweils an solchen Orten vorgesehen sind, auf die lehrmäßig festgelegte und exakt definierte Akupunkturpunkte der Körperoberfläche eines auf dem Kissen (6) ruhenden Kopfes in Bauch- und/oder Rücken- und/oder Seitenlage zu liegen kommen.

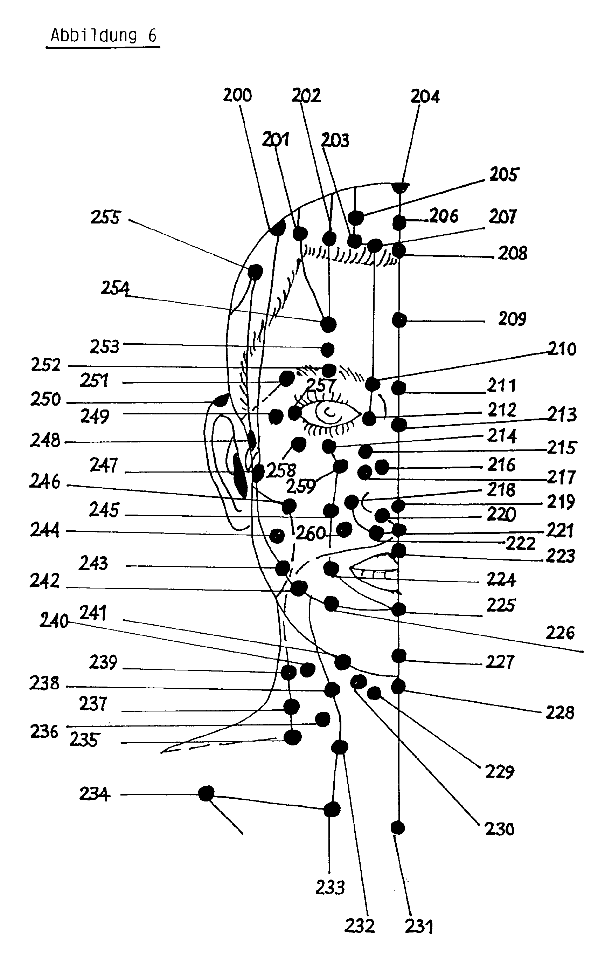

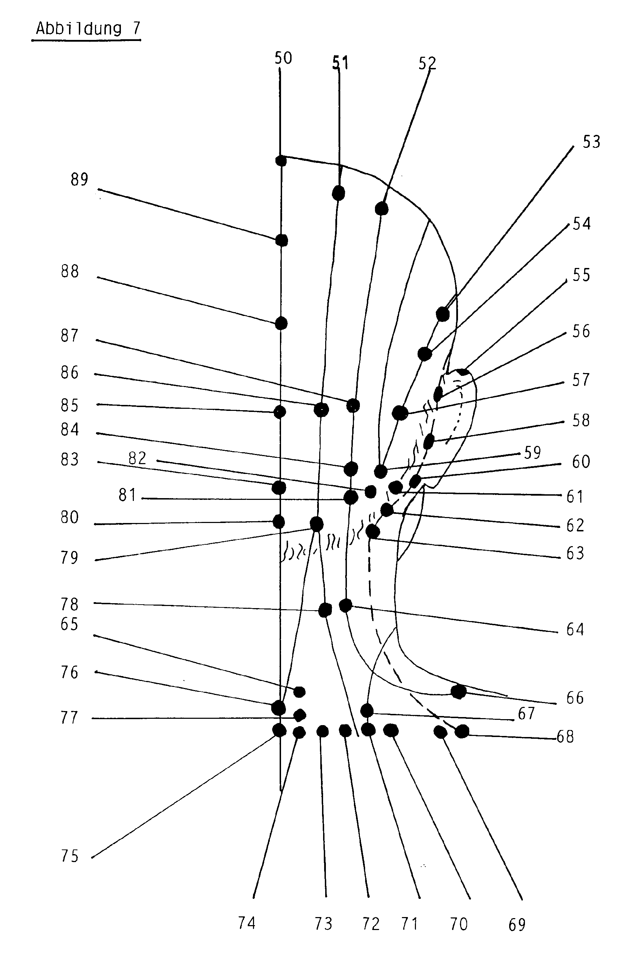

Welche Akupunkturpunkte - und in Verbindung damit - welche Orte des Kissens für die Anbringung von Stimulationsvorrichtungen (3) im einzelnen besonders geeignet sind, ergibt sich aus den Abbildungen 6 bis 8 sowie den zugehörigen Tabellen 1 bis 3.

Dort sind die im Bereich des Gesichtsschädels und des Hinterkopfes liegenden sowie im seitlichen Kopfbereich befindlichen Akupunkturpunkte graphisch dargestellt, in ihrer Beziehung zum Meridianverlauf zu sehen und nach der im deutschen Sprachraum üblichen Nomenklatur benannt.

Der einem bestimmten Akupunkturpunkt entsprechende Anbringungsort einer Stimulationsvorrichtung (3) auf dem Kissen (6) ergibt sich aus einer Projektion dieses Akupunkturpunktes auf den nächstliegendenden Bereich der Oberfläche der mittleren, muldenförmigen Aussparung (1) des Kissens (6) und/oder der Nacken- beziehungsweise Kinnauflage (2) und/oder des Seitenteils (18).

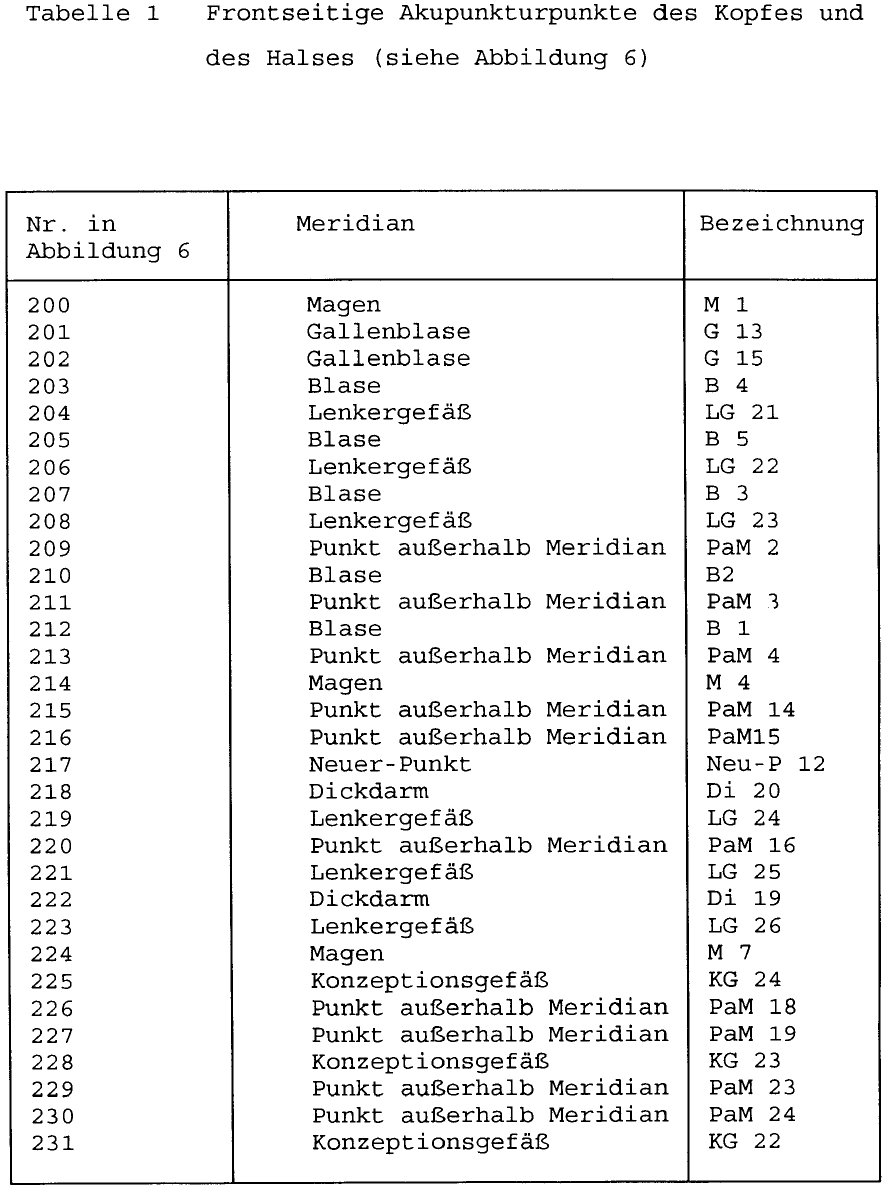

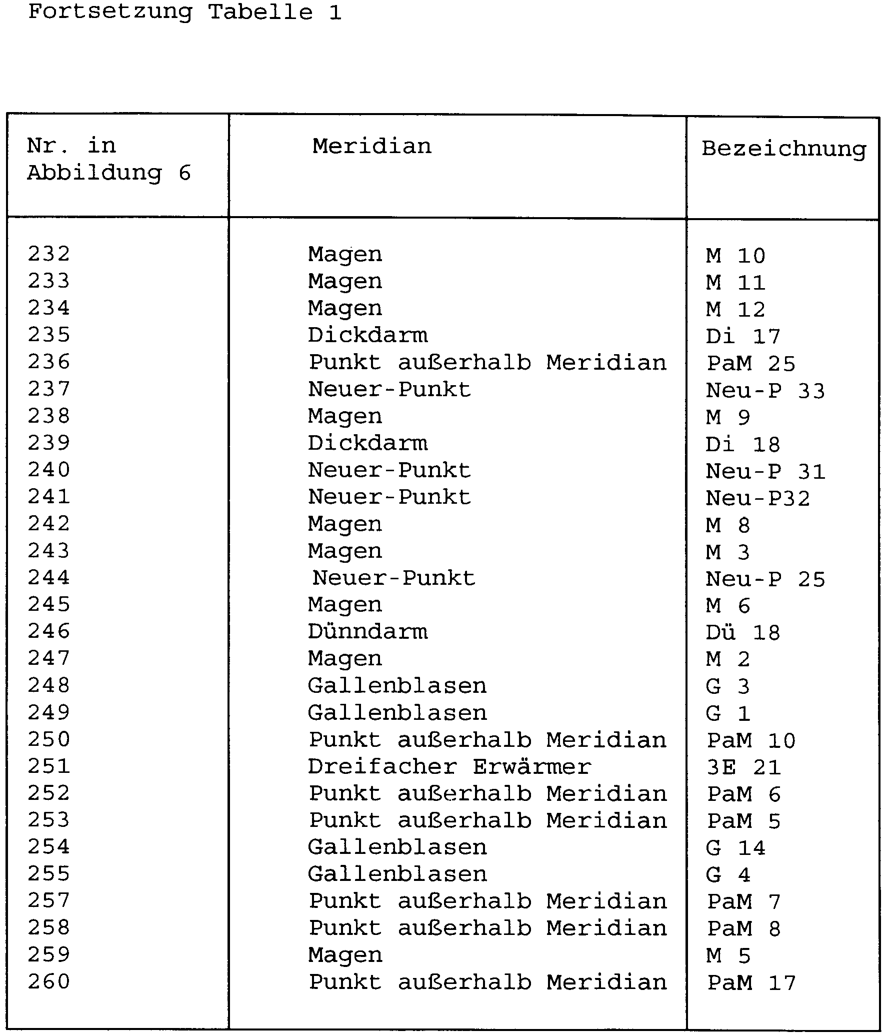

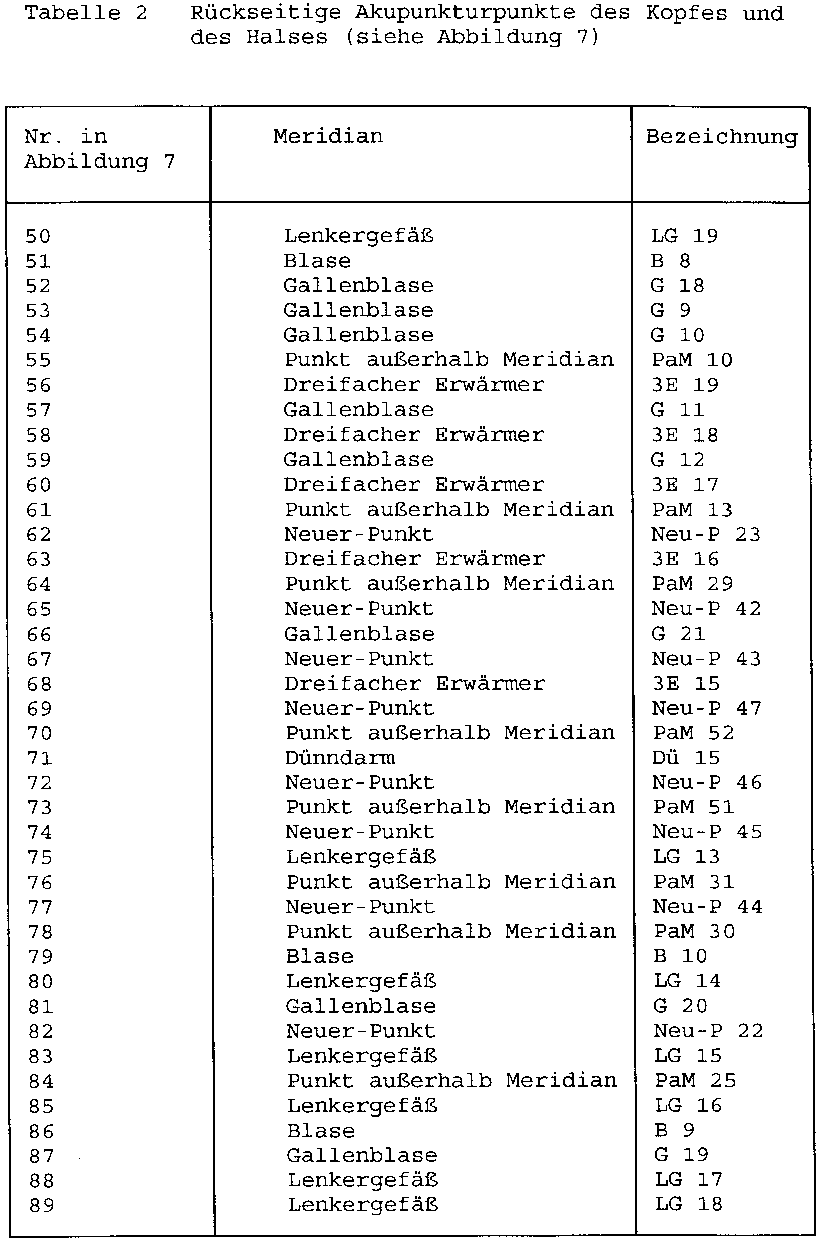

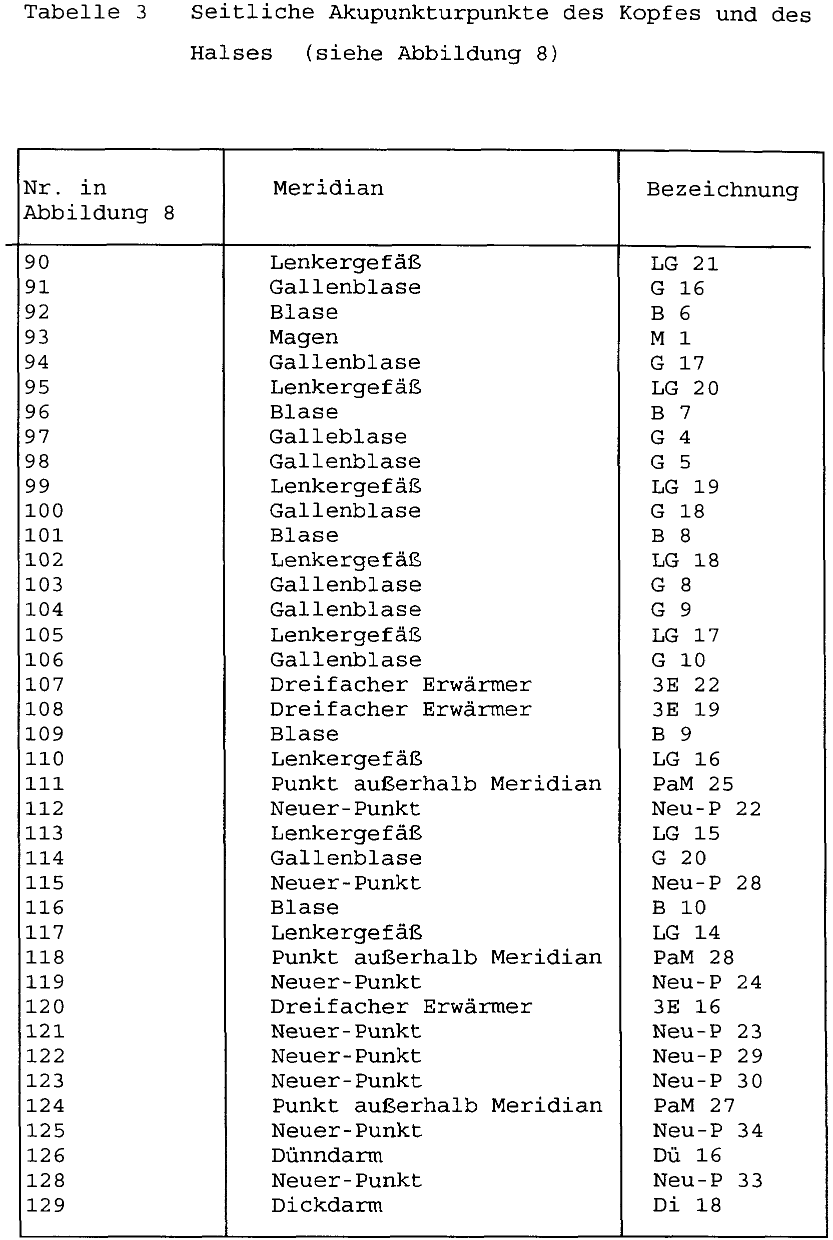

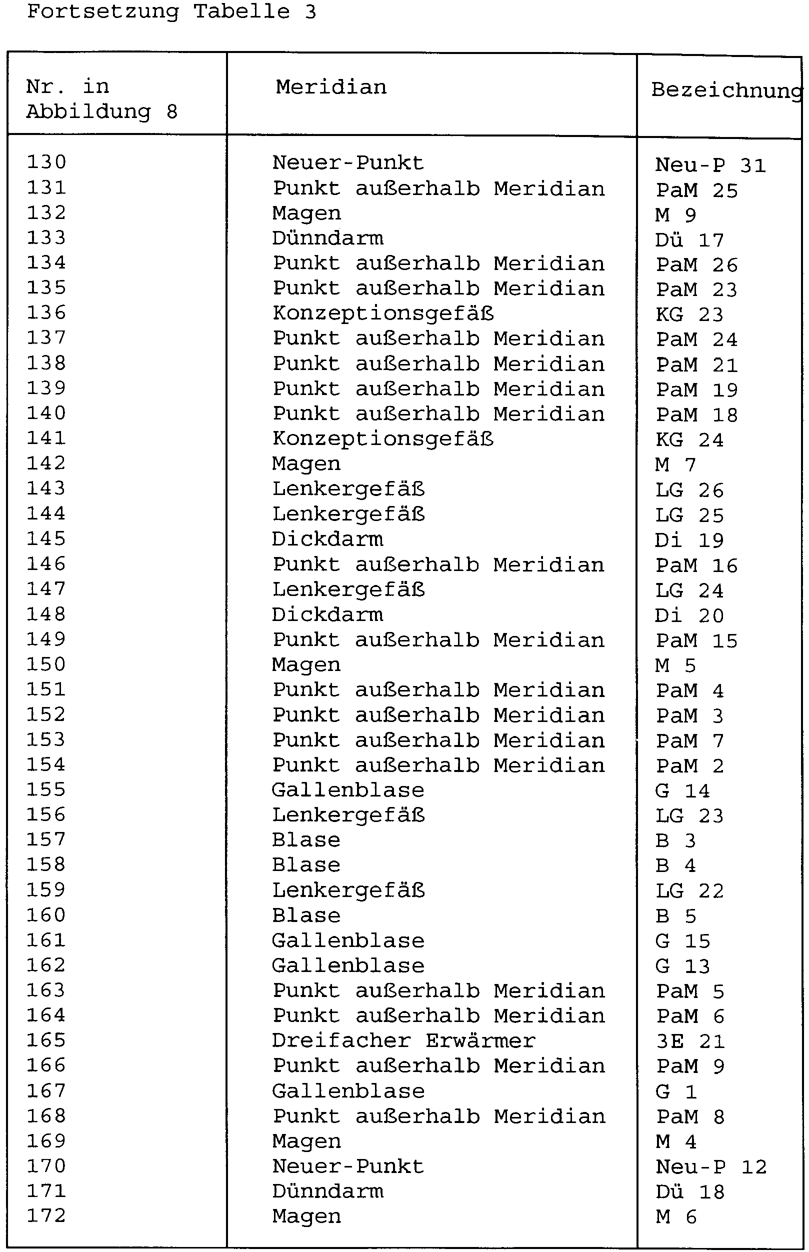

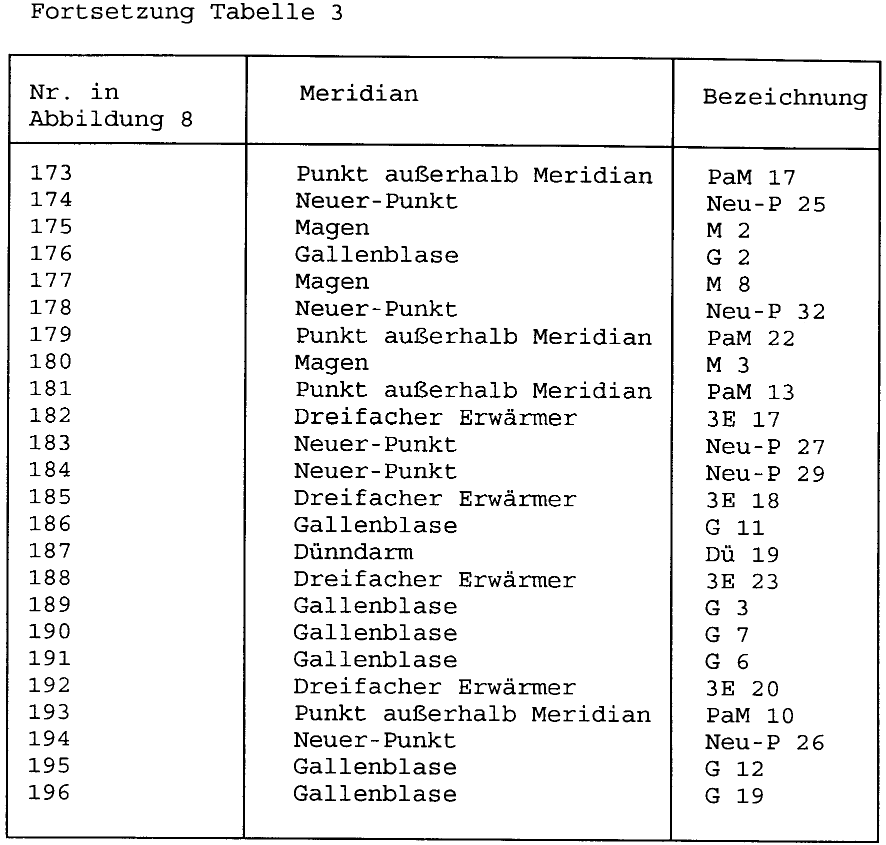

Grundsätzlich kommen für die Ermittlung der Anbringungsorte der Stimulationsvorrichtungen (3) auf der Kissenoberfläche alle Akupunkturpunkte im Kopf- und Halsbereich in Betracht, insbesondere auch die außerhalb eines Meridianes liegenden Punkte (PaM's) und die erst neu gefundenen, therapeutisch wirksamen Punkte (Neu-P's).

Ein Großteil der hier interessierenden Akupunkturpunkte ist beispielsweise in "Punkte und Regeln der neuen chinesischen Akupunktur", G. König, I. Wancura, 1990, Verlag für medizinische Wissenschaften, Wien, angegeben und dort in übersichtliche Beziehung zum menschlichen Skelett gesetzt. Durch die Zuordenbarkeit aller Akupunkturpunkte zu ertastbaren, eindeutigen Stellen am Skelett ergibt sich für die Anbringung der einzelnen Stimulationsvorrichtung (3) jeweils ein eindeutig definierter Ort. Die exakte Auffindung des Akupunkturpunktes im Einzelfalle wird dadurch erleichtert, daß alle Akupunkturpunkte im Vergleich zu den sie umgebenden Hautbereichen einen herabgesetzten elektrischen Hautwiderstand besitzen und deshalb mit Hilfe von elektronischen Leitfähigkeitsmessern mühelos und exakt lokalisiert werden können. Histologisch sind die Akupunkturpunkte durch eine Anhäufung rezeptiver Hautelemente gekennzeichnet.

Die gemäß den Abbildungen 6 bis 8 sowie den Tabellen 1 bis 3 vorzugsweise für die Ermittlung der Anbringungsorte der Stimulationsvorrichtungen (3) auf dem Kissen (6) geeigneten Akupunkturpunkte befinden sich insbesondere auf dem lehrmäßigen Dünndarm-Meridian (Dü), dem lehrmäßigen Blasen-Meridian (B), dem lehrmäßigen Dreifachen Erwärmer-Meridian (3E), dem lehrmäßigen Gallenblasen-Meridian (G), dem lehrmäßigen Lenkergefäß-Meridian (LG), dem lehrmäßigen Dickdarm-Meridian (Di), dem lehrmäßigen Magen-Meridian (M) und/oder dem lehrmäßigen Konzeptionsgefäß-Meridian (KG).

In besonders bevorzugten Ausführungsformen des erfindungsgemäßen Kissens befinden sich eine oder mehrere Stimulationsvorrichtungen (3) an solchen Orten auf der Kissenoberfläche, die einer Projektion der nachfolgend genannten Akupunkturpunkte auf diese entsprechen:
Dickdarm-Meridian (Di): 19, 20
Magen-Meridian (M): 1, 2, 3, 5, 7
Dünndarm-Meridian (Dü): 19
Blasen-Meridian (B): 2, 7, 10, 11
Dreifacher Erwärmer-Meridian (3E): 23
Gallenblasen-Meridian (G): 2, 14
Lenkergefäß-Meridian (LG): 14, 15, 19, 22, 23, 25
Konzeptionsgefäß-Meridian (KG): 24

Zur Übertragung der im Einzelfalle am Kopf oder Hals ertasteten oder anderweitig ermittelten individuellen Akupunkturpunkte auf die Oberfläche des Kissens (6) werden diese zum Beispiel mit einer geeigneten Markierungssubstanz am Kopf und/oder Hals markiert. Wird der Kopf nun auf das Kissen gelegt, übertragen sich diese Markierungen auf die Oberfläche des Kissens. Die auf diese Weise markierten Orte der Kissenoberfläche stellen die Anbringungsorte der Stimulationsvorrichtungen (3) dar. Als Mittel zur Markierung eignen sich beispielsweise Graphitpulver, gegebenenfalls mit einem Fluoreszenzfarbstoff angefärbtes Vaselin oder dergleichen.

Ein besonderer Vorteil des erfindungsgemäßen Kissens besteht darin, daß die Stimulationsvorrichtungen (3) beispielsweise selektiv nur an einigen, nach der Lehre der Akupunktur und/oder der Akupressur bewußt ausgewählten Akupunkturpunkten entsprechenden Orten auf der Oberfläche des Kissens (6) angebracht werden können.
Auf diese Weise ist es möglich, die Stimulationsvorrichtungen (3) eines erfindungsgemäßen Kissens so zu positionieren, daß das bestückte Kissen dazu geeignet ist, auf eine einzelne, definierte Störung des Wohlbefindens begünstigend einzuwirken. Für unterschiedliche Störungen des Wohlbefindens können demnach unterschiedlich mit Stimulationsvorrichtungen (3) bestückte erfindungsgemäße Kissen (6) bereitgestellt werden.

In einer Standardausführung des erfindungsgemäßen Kissens sind die Stimulationsvorrichtungen (3) an der Oberfläche des Kissens (6) durch ein Aufkleben, Aufnähen, Einnähen oder Einschweißen entsprechend einer bestimmten Indikation irreversibel angebracht.
In bevorzugten Ausführungsformen sind die Stimulationsvorrichtungen (3) jedoch auf der Oberfläche des Kissens im Bereich der mittleren muldenförmigen Aussparung (1) und/oder des Seitenteils (18) reversibel entfernbar und versetzbar angebracht.

Beispielsweise können die Stimulationsvorrichtungen (3) an Orten, die einer Projektion lehrmäßiger Akupunkturpunkte entsprechen, in loch- und/oder taschenförmige Aussparungen des Kissens (6) gesteckt werden. Alternativ hierzu können sie mittels eines am unteren Ende der Stimulationsvorrichtung (3) ausgebildeten Gewindeschaftes in das Kissen eingeschraubt werden. Schließlich ist es möglich, die Stimulationsvorrichtung (3) an der klebenden Unterseite eines beispielsweise kreisförmigen Pflasters vorzusehen und diese mit Hilfe des Pflasters auf dem Kissen zu fixieren.

Zur Zuordnung der Stimulationsvorrichtungen (3) zu Orten auf dem Kissen (6), die Projektionen lehrmäßig festgelegter Akupunkturpunkte auf die Kissenoberfläche entsprechen, können auf der Oberfläche der mittleren muldenförmigen Aussparung (1) und/oder der Nackenbeziehungsweise Kinnauflage (2) und/oder des Seitenteils (18) an den den Akupunkturpunkten entsprechenden Stellen jeweils ein oder mehrere Zuordnungsmittel vorgesehen werden.

Diese Zuordnungsmittel stellen beispielsweise gedruckte, erhabene oder versenkte Ziffern, Buchstaben oder eine Kombination hiervon dar. Alternativ hierzu können punkt-, strich- oder kreuzförmige Erhebungen, Versenkungen oder Druckmotive sowie ein gedrucktes, erhabenes oder versenktes, im wesentlichen gitterförmiges Koordinatennetz als Zuordnungsmittel zum Einsatz kommen. Gegebenenfalls sind Leuchtmittel wie Dioden oder Enden von Glasfaserlichtleitern zur besseren Kenntlichmachung der Akupunkturpunkten entsprechenden Orte vorgesehen. Auch die Verwendung von fluoreszierenden und/oder phosphoresziernden, gegebenenfalls unterschiedlich gefärbten Farbstoffen ist zur Kennzeichnung der Akupunkturpunkten entsprechenden Orte denkbar.

Besonders vorteilhaft ist im Falle des erfindungsgemäßen Kissens, daß eine einzige mittlere, muldenförmige Aussparung (1) zur zeitlich aufeinanderfolgenden Applikation von physikalischen Reizen auf den Hinterkopf, den Gesichtsschädel und den seitlichen Bereich des Kopfes ausreicht.
Durch drei beispielsweise durch unterschiedliche Farbgebung voneinander unterscheidbare Zuordnungsmittelsysteme für die Akupunkturpunkte des Hinterkopfes, des Gesichtsschädels und des seitlichen Kopfbereiches wird stets ein zielgerichtetes Versetzen der Stimulationsvorrichtungen (3) ermöglicht.

In einer Standardausführung verfügt das erfindungsgemäße Kissen (6) lediglich über eine mittlere, im wesentlichen muldenförmige Aussparung (1), die keine besondere Ausgestaltung aufweist.

Zur Steigerung der Bequemlichkeit während des Liegens in der Bauchlage besitzt das erfindungsgemäße Kissen (6) in bevorzugten Ausführungsformen jedoch eine besonders ausgestaltete, der Anatomie des Gesichtsschädels angepaßte, mittlere muldenförmige Aussparung (1).

Beispielsweise kann im scheitelseitigen Randbereich der mittleren, muldenförmigen Aussparung (1) ein in etwa an die Anatomie der Stirn angepaßter, flächiger Stützbereich (10) für die Stirn vorgesehen werden. Desweiteren kann eine längliche, der Anatomie der Nase angepaßte Aussparung (24) mittig und kinnseitig zur Stirnauflage (10) ausgebildet sein. Zur Vermeidung eines Druckes auf die Augen können beidseitig von der Nasen-Aussparung (24) Augenaussparungen (15) in die mittlere, muldenförmige Aussparung (1) eingearbeitet sein. Unterhalb der Augenaussparungen (15) ist es möglich, sich beidseitig seitlich erstreckende Jochbein-Auflagen (11) anzubringen. Der in Richtung des Rückens eines Benutzers weisende, obere Randabschnitt (2) der mittleren muldenförmigen Aussparung (1) kann zumindest etwas abgeflacht werden, so daß er die Funktion einer Kinnauflage (2) erfüllt.

Von besonderem Vorteil ist es, wenn die Stirnauflage (10) und die Jochbeinauflage (11) so breit ausgebildet sind, daß sie bei Rückenlage den Hinterkopf anschmiegend umfassen und wenn die Kinnauflage (2) entsprechend der Nakkenbreite eingemuldet ist.

Außerordentlich vorteilhaft ist es ferner, die mittlere muldenförmige Aussparung (1) mit einem mittig und zentral angeordneten, sich nach unten im wesentlichen trichterförmig erstreckenden Durchbruch (13) im Bereich von Mund und Nase zu versehen. Vorzugsweise fallen die Stirnauflage (10), die Jochbeinauflagen (11) und die Kinnauflage (2) in Richtung des Durchbruchs (13) bogenförmig ab.

Um die Atmung durch das erfindungsgemäße Kissen (6) in Bauchlage zu erleichtern, kann mindestens ein gegen ein Zusammendrücken gesicherter, durch das Kissen verlaufender Luftkanal (14) vorgesehen werden, welcher in den Durchbruch (13) einmündet. Zur Erleichterung der Atmung ist es weiterhin möglich, in der gegebenenfalls vorhandenen Bodenplatte (19) im Bereich des Durchbruchs (13) eine Öffnung (20) vorzusehen. Die Bodenplatte (19) kann an der Unterseite des Kissens (6) angebracht sein, um eine Arretierung des Kissens (6) zu ermöglichen und um den Luftkanal (14) freizuhalten und um dem Kissen (6) eine gewisse Steifigkeit zu verleihen.

Um eine möglichst weit in Richtung des Rückens eines Benutzers reichende Unterstützung des Nackens durch die Nackenauflage (2) zu erreichen, ist der obere Teil der rückenwärtigen, im wesentlichen senkrechten Außenwandung (16) des Kissens (6) im Schulter- und Nackenbereich vorzugsweise in Form eines Vorsprungs (17) in Richtung des Rückens vorgewölbt.

Zur Gewährleistung der Erhaltung einer physiologischen Halslordose in der Seitenlage können die sich seitlich neben der mittleren muldenfömigen Aussparung (1) erstreckenden Seitenteile (18) auf ihrer Oberseite mit muldenförmigen Vertiefungen (25) versehen werden, deren Abmessungen an die Anatomie des seitlichen Kopfbereichs angepaßt sind und insbesondere die Ohren entlasten.

Vorzugsweise ist die Oberseite der Seitenteile (18) bei seitlicher Betrachtung in Richtung des Scheitels des Betrachters zumindest etwas abfallend geneigt. Die Höhe der Seitenteile (18) entspricht im wesentlichen der Breite des Schulterblattes.

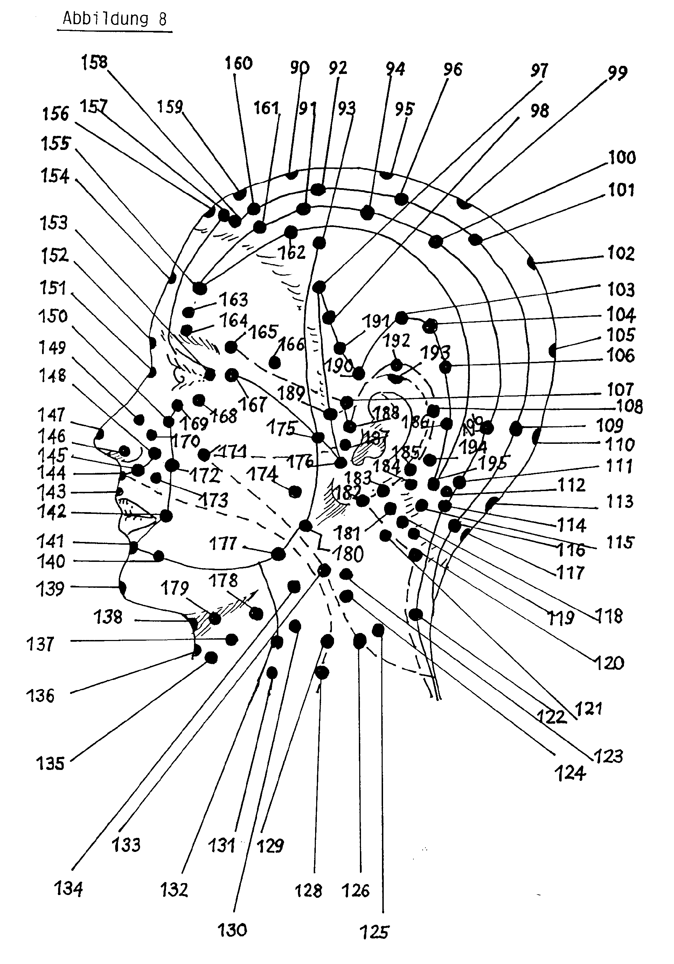

Wie bereits ausgeführt, ist es selbstverständich, daß sich auch auf diesen Seitenteilen (18) des Kissens (6) eine oder mehrere Stimulationsvorrichtungen (3) befinden können. Deren Anbringungsorte entsprechen vorzugsweise Projektionen der auf der seitlichen Kopfhälfte befindlichen Akupunkturpunkte auf die Oberfläche der Seitenteile (18) (siehe Abbildung 8; Tabelle 3).

Bevorzugte Ausführungsformen des erfindungsgemäßen Kissens weisen in der Draufsicht eine leicht in Richtung der Schultern gebogene, längliche Form auf.
Hierdurch wird einerseits einer Kippbewegung des Kissens (6) in Richtung des Rückens des Benutzers entgegengewirkt. Andererseits erfährt das in der Seitenlage auf dem Seitenteil (18) aufliegende Kinn durch den in Richtung der Schulter vorgebogenen seitlichen Abschnitt des Kissens (6) eine besonders wirkungsvolle Unterstützung.

Zur Herstellung des erfindungsgemäßen Kissens (6) eignet sich insbesondere geschlossen- oder offenporiger, natürlicher oder synthetischer Schaumstoff.

Die Auflageflächen im Bereich der mittleren, muldenförmigen Aussparung (1) und/oder der Halsabstützung (2) und/oder der muldenförmigen Vertiefungen (25) auf den Seitenteilen (18) können zur Verbesserung des Kontaktes mit der jeweiligen Hautpartie zusätzlich Vorrichtung zur Anpassung an die individuellen anatomischen Gegebenheiten aufweisen.
Beispielsweise kann eine doppelwandige Ausführung dieser Bereiche gewählt werden, wobei zwischen die Wände ein Gel, eine Flüssigkeit oder ein Gas eingeführt wird.
Es ist auch denkbar, zwischen derartigen Wänden beispielsweise bei Normaldruck gegeneinander verschiebbare kleine Styroporkugeln vorzusehen, die beim Anlegen eines Unterdruckes nicht mehr gegeneinander verschiebbar sind und somit auch eine hohe Haltekraft entfalten, die sich bei der Verwendung des erfindungsgemäßen Kissens durch Kinder positiv bemerkbar macht.

Zusammenfassend ist festzustellen, daß das erfindungegemäße Kissen zu zahlreichen Vorteilen gegenüber der herkömmlichen Nadel-Akupunktur und der Pflaster-Nadel-Akupunktur führt.

Mit Hilfe des erfindungsgemäßen Kissens, dessen Stimulationsvorrichtungen in der Regel nicht nadelförmig sind, werden Einstichschmerzen und in Verbindung damit Verkrampfungen und Kollapszustände vermieden. Die Gefahr der Entzündung einer Einstichstelle und eine Infektionsgefahr sind ausgeschlossen. Auch Unverträglichkeitsreaktionen gegenüber dem Nadelmaterial oder dem Pflastermaterial scheiden aus. Ein gegebenenfalls entstellendes Ausrasieren des Haupthaares zum Zwecke der Verabreichung physikalischer Reize ist überflüssig. Ein mühsames und schmerzhaftes Entfernen von Pflastermaterial aus den Haaren entfällt. Während des Schlafes geht von dem erfindungsgemäßen Kissen weder eine Verletzungs- noch eine Verunreinigungsgefahr aus. Da geringe Bewegungen des Kopfes und/oder Nackens während einer Anwendung zu einer in den meisten Fällen gewünschten, zusätzlichen, sanften Stimulierung des jeweiligen Akupunkturpunktes durch die in der Regel zumindest etwas über die Kissenoberfläche ragende Stimulationsvorrichtung (3) führen, besteht keine Notwendigkeit für ein Redeverbot während der Behandlung.

Das erfindungsgemäße Kissen erfreut sich deswegen insbesondere bei Kindern großer Beliebtheit. Sind die für den jeweiligen Patienten entscheidenden Anbringungsorte der Stimulationsvorrichtungen (3) einmal festgelegt, so können zahlreiche Sitzungen ohne eine erneute, zeitintensive Ermittlung dieser Orte nachfolgen. Dies führt zu erheblichen Einsparungen der Arbeitszeit des Behandlers und damit zu einer Verringerung der durch die Krankenkassen zu erstattenden Behandlungskosten. Für den Benutzer ergibt sich überdies der Vorteil, daß er das erfindungsgemäße Kissen jederzeit und zu Hause verwenden kann.

Abschließend wird angemerkt, daß in besonders bevorzugten Ausführungsformen des erfindungsgemäßen Kissens mindestens ein Lautsprecher in mindestens einem der Seitenteile (18) enthalten ist, mit dessen Hilfe dem Kissenbenutzer harmonische Musik zugespielt wird. Es hat sich gezeigt, daß hierdurch in kürzester Zeit ein besonders tiefer Entspannungszustand erreicht wird, der den Behandlungserfolg günstig beeinflußt.

## Patentansprüche

1. Kopfkissen aus nicht wesentlich verformbarem Material mit einer mittleren muldenförmigen Aussparung (1) zur Aufnahme und Abstützung des Kopfes bei Rückenlage und/oder Bauchlage und/oder Seitenlage, welche im wesentlichen entsprechend der Anatomie des Hinterkopfes und/oder der Gesichtshälfte und/oder des seitlichen Kopfbereiches geformt ist, **dadurch gekennzeichnet, daß** auf der Oberfläche der muldenförmigen Aussparung (1) und/oder im Bereich der Nacken- beziehungsweise Kinnauflage (2) und/oder auf der Oberseite mindestens eines Seitenteils ((18) des Kissens (6) mindestens eine feststehende Stimulationsvorrichtung (3) zur Ausübung von physikalischen Reizen wie von magnetischen Reizen und/oder elektrischen Reizen und/oder Druckreizen und/oder thermischen Reizen und/oder Lichtreizen vorgesehen ist.

2. Kissen nach Anspruch 1, **dadurch gekennzeichnet, daß** die Stimulationsvorrichtung (3) ein kleiner Magnet ist, der gegenüber der Oberfläche des Kissens erhaben oder in das Kissen integriert ist und eine in Richtung des Kopfes abgerundete, spitze oder nadelförmige Form aufweist.

3. Kissen nach Anspruch 2, **dadurch gekennzeichnet, daß** der Magnet eine magnetische Feldstärke im Bereich von 400 bis 1000 Gauß, vorzugsweise von 500 bis 900 Gauß, insbesondere von 600 bis 800 Gauß aufweist, ein Permanentmagnet ist oder durch eine stromdurchflossene Spule mit Eisenkern verkörpert wird, kugelförmig, kreisförmig, oval, länglich oder stabförmig ist und aus Ferrit, aus nickel-, kobalt- und/oder eisenhaltigen Stoffen oder aus keramischen oder metallischen Magnetwerkstoffen hergestellt ist.

4. Kissen nach Anspruch 1, **dadurch gekennzeichnet, daß** die Stimulationsvorrichtung (3) ein in Richtung des Kopfes spitzes, nadelförmiges oder abgerundetes massives Druckelement und/oder ein Druckelement ist, welches einen zielgerichteten Wasser- oder Luftstrahl freisetzt und/oder ein Druckelement ist, das Schallwellen abgibt, wobei jedes dieser Druckelemente die Oberfläche des Kissens (6) überragt oder in dieses integriert ist und während des Aufliegens des Kopfes einen im wesentlichen punktförmigen Druck auf diesen und/oder den Halsbereich ausübt.

5. Kissen nach Anspruch 1, **dadurch gekennzeichnet, daß** die Stimulationsvorrichtung (3) eine in Richtung des Kopfes abgerundete, spitze oder nadelförmige Laserstrahlen ausstrahlende Vorrichtung ist, welche die Oberfläche des Kissens (6) überragt oder in dieses integriert ist und während des Aufliegens des Kopfes im wesentlichen punktförmige Licht- und/oder thermische Reize auf diesen und/oder den Halsbereich ausübt.

6. Kissen nach Anspruch 1, **dadurch gekennzeichnet, daß** die Stimulationsvorrichtung (3) eine in Richtung des Kopfes abgerundete, spitze oder nadelförmige farbiges Licht ausstrahlende Vorrichtung ist, welche die Oberfläche des Kissens (6) überragt oder in dieses integriert ist und während des Aufliegens des Kopfes im wesentlichen punktförmige Lichtreize auf diesen und/oder den Halsbereich ausübt.

7. Kissen nach Anspruch 1, **dadurch gekennzeichnet, daß** die Stimulationsvorrichtung (3) ein kleines, in Richtung des Kopfes abgerundetes, spitzes oder nadelförmiges Heiz- und/oder Kühlelement oder ein temperierte Luft oder Wasser zielgerichtet abgebendes Element ist, welches die Oberfläche des Kissens (6) überragt oder in dieses integriert ist und während des Aufliegens des Kopfes eine punktuelle Erwärmung oder Abkühlung bewirkt.

8. Kissen nach Anspruch 1, **dadurch gekennzeichnet, daß** die Stimulationsvorrichtung (3) eine kleine, in Richtung des Kopfes abgerundete, spitze oder nadelförmige Elektrode ist, welche die Oberfläche des Kissens (6) überragt oder in dieses integriert ist und während des Aufliegens des Kopfes punktuell niederfrequente Wechselströme weiterleitet.

9. Kissen nach einem der Ansprüche 1 bis 8, **dadurch gekennzeichnet, daß** die Gesamtheit aller auf dem Kissen vorgesehenen Stimulationsvorrichtungen (3) eine Kombination von einem oder mehreren Magneten, Druckelementen, Heiz- und/oder Kühlelementen, Elektroden, Vorrichtungen zur Ausstrahlung von Laserstrahlen und/oder von Vorrichtungen zur Ausstrahlung von farbigem Licht darstellt.

10. Kissen nach einem der Ansprüche 1 bis 9, **dadurch gekennzeichnet, daß** die Stimulationsvorrichtungen (3) in der muldenförmigen Aussparung (1) und/oder in der Nackenbeziehungsweise Kinnauflage (2) und/oder auf der Oberseite mindestens eines Seitenteils (18) des Kissens (6) jeweils an solchen Orten vorgesehen sind, auf die lehrmäßig festgelegte und gegenüber dem Skelett exakt definierte Akupunkturpunkte der Oberfläche eines auf dem Kissen (6) ruhenden Kopfes und/oder Nackens in Bauch- und/oder Rücken- und/oder Seitenlage zu liegen kommen, wobei die Anzahl und die Positionen der möglichen Anbringungsorte der Stimulationsvorrichtungen (3) auf dem Kissen (6) der Anzahl und den Positionen aller Akupunkturpunkte im Kopf- und Halsbereich entsprechen und wobei diese Akupunkturpunkte gegenüber den sie umgebenden Hautbereichen einen herabgesetzten elektrischen Hautwiderstand besitzen und histologisch eine Anhäufung rezeptiver Hautelemente aufweisen.

11. Kissen nach Anspruch 10, **dadurch gekennzeichnet, daß** die Stimulationsvorrichtungen (3) in der muldenförmigen Aussparung (1) und/oder der Nackenauflage (2) und/oder mindestens einem Seitenteil (18) an einem oder mehreren Orten vorgesehen sind, die einer Projektion von definierten Akupunkturpunkten auf das Kissen entsprechen, welche sich auf dem lehrmäßigen Dünndarm-Meridian (Dü), dem lehrmäßigen Blasen-Meridian (B), dem lehrmäßigen Dreifachen-Erwärmer-Meridian (3E), dem lehrmäßigen Gallenblasen-Meridian (G), dem lehrmäßigen Lenkergefäß-Meridian (LG), dem lehrmäßigen Dickdarm-Meridian (Di), dem lehrmäßigen Magen-Meridian (M) und dem lehrmäßigen Konzeptionsgefäß-Meridian (KG) einer auf dem Kissen (6) ruhenden Person in Bauch- und/oder Seiten- und/oder Rückenlage befinden.

12. Kissen nach Anspruch 10 oder 11, **dadurch gekennzeichnet, daß** die Stimulationsvorrichtungen (3) in der muldenförmigen Aussparung (1) und/oder der Nackenbeziehungsweise Kinnauflage (2) und/oder auf mindestens einem Seitenteil (18) des Kissens (6) an Orten vorgesehen sind, die einer Projektion der nachfolgend genannten, lehrmäßig festgelegten Akupunkturpunkte auf die Oberfläche der muldenförmigen Aussparung (1) und/oder der Nackenauflage (2) und/oder die Oberseite des Seitenteils (18) entsprechen:
Dickdarm-Meridian (Di) : 19, 20
Magen-Meridian (M) : 1, 2, 3, 5, 7
Dünndarm-Meridian (Dü) : 19
Blasen-Meridian (B) : 2, 7, 10, 11
Dreifacher Erwärmer-Meridian (3E) : 23
Gallenblasen-Meridian (G): 2, 14
Lenkergefäß-Meridian (LG) : 14, 15, 19, 22, 23, 25
Konzeptionsgefäß-Meridian (KG) : 24

13. Kissen nach einem der Ansprüche 1 bis 12, **dadurch gekennzeichnet, daß** die Stimulationsvorrichtungen (3) auf der Oberfläche des Kissens reversibel entfernbar und versetzbar angebracht sind.

14. Kissen nach einem der Ansprüche 10 bis 13, **dadurch gekennzeichnet, daß** die Stimulationsvorrichtungen (3) lediglich an den Positionen des Kissens (6) angebracht sind, die denjenigen Akupunkturpunkten entsprechen, welche nach der festgeschriebenen Lehre der Akupunktur und/oder der Akupressur zu stimulieren sind, um auf eine einzelne, definierte Störung des Wohlbefindens begünstigend einzuwirken.

15. Kissen nach einem der Ansprüche 10 bis 14, **dadurch gekennzeichnet, daß** die Stimulationsvorrichtungen (3) an Orten, die lehrmäßigen Akupunkturpunkten entsprechen, in loch- oder taschenförmige Aussparungen des Kissens (6) gesteckt werden und/oder mittels eines am unteren Ende der Stimulationsvorrichtung (3) ausgebildeten Gewindeschaftes eingeschraubt werden und/oder an der klebenden Unterseite eines kreisförmigen Pflasters befindlich aufgeklebt werden.

16. Kissen nach einem der Ansprüche 1 bis 15, **dadurch gekennzeichnet, daß** es eine längliche Form mit einer mittleren Aussparung (1) aufweist, die zur Aufnahme des Kopfes in Bauchlage mit an die Anatomie der Gesichtshälfte angepaßten Stützbereichen für Stirn (10), Jochbeine (11) und Kinn (2) versehen ist, wobei die Stirnauflage (10) und die Jochbeinauflage (11) so breit ausgebildet sind, daß sie bei Rückenlage den Hinterkopf anschmiegend umfassen und wobei die Kinnauflage (2) entsprechend der Nackenbreite eingemuldet ist und in Richtung Stirnauflage (10) beziehungsweise zum Durchbruch (13) hin bogenförmig abfällt, daß die Stirnauflage (19) gegenüber der Kinnauflage (2) zumindest etwas tiefer liegt und daß die mittlere, muldenförmige Aussparung (1) im Bereich von Mund und Nase einen sich nach unten erstreckenden Durchbruch (13) aufweist.

17. Kissen nach Anspruch 16, **dadurch gekennzeichnet, daß** in den sich nach unten erstreckenden Durchbruch (13) der mittleren muldenförmigen Aussparung (1) mindestens ein in das Kissen eingearbeiteter, gegen ein Zusammendrücken gesicherter Luftkanal (14) einmündet und/oder für die Nase eine mittig von der Stirnauflage (10) in Richtung der Kinnauflage (2) ausgehende, längliche Nasenaussparung (24) vorgesehen ist und/oder die mittlere muldenförmige Aussparung (1) im Bereich der aufliegenden Augen Vertiefungen (15) zur Verminderung eines Druckes auf die Augen aufweist und/oder die rückenseitige, im wesentlichen senkrecht verlaufende Fläche (16) des Kissens (6) im Bereich der Nacken- beziehungsweise Kinnauflage (2) und/oder im seitlichen Schulterbereich (18) zumindest etwas in Form eines Vorsprungs (17) in Richtung des Rückens vorgewölbt ist.

18. Kissen nach Anspruch 16 oder 17, **dadurch gekennzeichnet, daß** die sich seitlich neben der mittleren muldenförmigen Aussparung (1) erstreckenden Seitenteile (18) nach Höhe, Abmessung und Neigung so gestaltet sind, daß der Kopf in Seitenlage des Körpers unter Erhaltung der physiologischen Halslordose aufliegt.

19. Kissen nach einem der Ansprüche 16 bis 18, **dadurch gekennzeichnet, daß** die Unterseite des Kissens (6) von einer Platte (18) gebildet wird, die das Kissen (6) geschlossen nach unten begrenzt oder zumindest im Bereich des Durchbruches (13) eine Öffnung (20) aufweist.

20. Kissen nach einem der Ansprüche 16 bis 19, **dadurch gekennzeichnet, daß** es in der Draufsicht eine leicht in Richtung der Schultern gebogene, längliche Form aufweist.

21. Kissen nach einem der vorhergehenden Ansprüche, **dadurch gekennzeichnet, daß** auf der Oberfläche der mittleren muldenförmigen Aussparung (1) und/oder der Nacken- beziehungsweise Kinnauflage (2) und/oder auf der Oberfläche mindestens eines Seitenteils (18) an den den jeweiligen Akupunkturpunkten entsprechenden Stellen ein oder mehrere Zuordnungsmittel vorgesehen sind zur Zuordnung von Stimulationsvorrichtungen (3) zu diesen Orten auf dem Kissen (6), die Projektionen der lehrmäßig festgelegten Akupunkturpunkte auf das Kissen (6) entsprechen, wobei mehrere, voneinander unterscheidbare Zuordnungsmittel-Systeme jeweils für die in Bauch-, Rükken- oder Seitenlage anzusprechenden Akupunkturpunkte auf der Oberfläche der mittleren muldenförmigen Aussparung (1) und/oder der Nacken-beziehungsweise Kinnauflage (2) und/oder des Seitenteils (18) anbringbar sind.

22. Kissen nach Anspruch 21, **dadurch gekennzeichnet, daß** das Zuordnungsmittel (21) mindestens eine gedruckte, erhabene oder versenkte Ziffer, ein Buchstabe oder eine Kombination hiervon ist und/oder mindestens eine punkt, strich- oder kreuzförmige Erhebung, Versenkung oder ein Druckmotiv ist und/oder ein gedrucktes, erhabenes oder versenktes, im wesentlichen gitterförmiges Koordinatennetz ist.

23. Kissen nach einem der vorhergehenden Ansprüche, **dadurch gekennzeichnet, daß** dessen Körper aus geschlossen- oder offenporigem, natürlichem oder synthetischem Schaumstoff hergestellt ist und/oder die Kopf- und Halsauflagebereiche des Kissens (6) doppelwandig augebildet sind, wobei zwischen die Wände ein Gel, eine Flüssigkeit oder ein Gas eingeführt wird oder wobei zwischen diese Wände kleine Feststoffkugeln eingebracht werden, die durch Anlegen eines Unterdruckes daran gehindert werden, gegeneinander verschiebbar zu sein.

24. Vorrichtung nach einem der vorhergehenden Ansprüche, **dadurch gekennzeichnet, daß** sie mindestens in einem der Seitenteile (18) einen Lautsprecher zur Zuspielung von entspannender Musik umfaßt.
